# EUROPEAN PATENT SPECIFICATION

(11) **EP 0 567 576 B1**
(45) Date of publication and mention of the grant of the patent: **04.01.1995**
(21) Application number: 92904687.8
(22) Date of filing: 15.01.1992
(51) Int. Cl.: A61K 38/20, C12N 15/24

(54) **TREATMENT OF NEOPLASTIC DISEASE WITH INTERLEUKIN-10**
BEHANDLUNG VON NEOPLASTISCHEN KRANKENHEITEN MIT INTERLEUKIN-10
TRAITEMENT DE MALADIE NEOPLASTIQUE A L'INTERLEUKINE 10

(30) Priority: 16.01.1991 US 641347
(43) Date of publication of application: 03.11.1993
(73) Proprietor: SCHERING CORPORATION, Kenilworth New Jersey 07033 (US)
(72) Inventor: VIEIRA, Paulo, J., M., Palo Alto, California 94303 (US); MOORE, Kevin, W., Palo Alto, CA 94306 (US)
(74) Representative: Ritter, Stephen David
(86) International application number: US9200066
(87) International publication number: WO9212725

(56) References cited:
- EP-A- 0 405 980
- WO-A-91/09127
- The Journal of Immunology, vol.145, n 12, 15 Dezember 1990 ( Baltimore, USA), I.A.MacNEIL et al. pages 4167-4173, see the whole article
- Journal of Experimental Medicine, vol. 172, no. 6, Dec. 1990 (New York, US), NING FEI et al. pages 1625-1631, see the whole article

## Description

The invention relates generally to compositions for treating neoplasms, or cancers, in humans, and more particularly, to the use of interleukin-10 (IL-10) For the manufacture of compositions for treating cancers.

Immunologic approaches to cancer therapy are based on the notion that cancer cells have somehow evaded the body's defenses against aberrant or foreign cells and molecules, and that these defenses might be therapeutically stimulated to regain the lost ground; e.g. pgs. 623-648 in Klein, Immunology (Wiley-Interscience, New York, 1982). The recent observations that various immune effectors can directly or indirectly inhibit tumor growth has led to renewed interest in this approach to cancer therapy: e.g. Herberman, Concepts Immunopathol., Vol. 1, pgs. 96-132 (1985) (natural killer cells resist tumor cell growth); Rosenberg et al., Ann. Rev. Immunol., Vol. 4, pgs. 681-709 (1988) (clinical use of IL-2-activated killer cells to treat cancer); Ralph et al., J. Exp. Med., Vol. 167, pgs. 712-717 (1988) (tumoricidal activity by macrophages stimulated by lymphokines), Tepper et al., Cell, Vol. 57, pgs. 503-512 (1989) (IL-4 has anti-tumor activity), M. Cohen, "Lymphokines and Tumor Immunity," pgs. 237-253 in S. Cohen, ed. Lymphokines and the Immune Response (CRC Press, Boca Raton, 1990), and the like.

The invention relates to the use of interleukin-10 (IL-10) to reduce and/or prevent the growth of tumors. The invention also includes pharmaceutical compositions comprising interleukin-10. Preferably, the interleukin-10 of the invention is selected from the group consisting of the mature polypeptides having the open reading frames that are defined by the amino acid sequences given in SEQ. ID. NOS. 1 and 2 herein (all SEQ. IDs. are given immediately before the Claims), wherein the standard three letter abbreviation is used to indicate L-amino acids, starting from the N-terminus. These two forms of IL-10 are sometimes referred to as human IL-10 (hIL-10 or human cytokine synthesis inhibitory factor) and viral IL-10 (vIL-10 or BCRF1), respectively: e.g. Moore et al., Science, Vol. 248, pgs. 1230-1234 (1990); Vieira et al., Science, Vol. 88, pgs. 1172-1176 (1991); Fiorentino et al., J. Exp. Med., Vol. 170, pgs. 2081-2095 (1989); Hsu et al., Science, Vol. 250, pgs. 830-832 (1990). More preferably, the mature IL-10 used in the method of the invention is selected from the group consisting of the mature polypeptides having the open reading frames that are defined by the amino acid sequences given in SEQ. ID. NOS. 3 and 4 herein.

Figure 1 is a diagrammatic representation of the mammalian expression vector pcD(SRα).

Figure 2 is a diagrammatic representation of the bacterial expression vector TRP-C11.

Figure 3 shows plasmid pGSRG carrying the open reading frame (ORF) of mouse IL-10, viral IL-10, or human IL-10 inserted into its *Xho* I restriction site; it also shows the sequence of the RBS-ATG-polylinker regions of the final construction (called TAC-RBS).

### DETAILED DESCRIPTION OF THE INVENTION

The invention is directed to the use of IL-10 in the manufacture of a medicament to prevent and/or reduce the growth of cancers. IL-10 for use in the invention is selected from the group of mature polypeptides encoded by the open reading frames defined by the cDNA inserts of pH5C, pH15C, and pBCRF1(SRα), which are deposited with the American Type Culture Collection (ATCC), Rockville, Maryland, under accession numbers 68191, 68192, and 68193, respectively.

A wide range of single-cell and multicellular expression systems (i.e. host-expression vector combinations) can be used to produce the polypeptides of the invention. Possible types of host cells include, but are not limited to, bacterial, yeast, insect, mammalian, and the like. Many reviews are available which provide guidance for making choices and/or modifications of specific expression systems, e.g. to name a few, de Boer and Shepard, "Strategies for Optimizing Foreign Gene Expression in Escherichia coli," pgs. 205-247, in Kroon, ed. Genes: Structure and Expression (John Wiley & Sons, New York, 1983), review several *E. coli* expression systems; Kucherlapati et al., Critical Reviews in Biochemistry, Vol. 16, Issue 4, pgs. 349-379 (1984), and Banerji et al., Genetic Engineering, Vol. 5, pgs. 19-31 (1983) review methods for transfecting and transforming mammalian cells; Reznikoff and Gold, eds., Maximizing Gene Expression (Butterworths, Boston, 1986) review selected topics in gene expression in *E. coli*, yeast, and mammalian cells; and Thilly, Mammalian Cell Technology (Butterworths, Boston, 1986) reviews mammalian expression systems. Likewise, many reviews are available which describe techniques and conditions for linking and/or manipulating specific cDNAs and expression control sequences to create and/or modify expression vectors suitable for use with the present invention, e.g. Sambrook et al. (cited above).

An *E. coli* expression system is disclosed by Riggs in U.S. Patent 4,431,739, which is incorporated by reference. A particularly useful prokaryotic promoter for high expression in *E. coli* is the tac promoter, disclosed by de Boer in U.S. Patent 4,551,433. Secretion expression vectors are also available for *E. coli* hosts. Particularly useful are the pIN-III-ompA vectors, disclosed by Ghrayeb et al., in EMBO J., Vol. 3, pgs. 2437-2442 (1984), in which the cDNA to be transcribed is fused to the portion of the *E. coli* OmpA gene encoding the signal peptide of the ompA protein which, in turn, causes the mature protein to be secreted into the periplasmic space of the bacteria. U.S. Patents 4,336,336 and 4,338,397 also disclose secretion expression vectors for prokaryotes.

Numerous strains of bacteria are suitable hosts for prokaryotic expression vectors including strains of *E. coli*, such as W3110 (ATCC No. 27325), JA221, C600, ED767, DH1, LE392, HB101, X1776 (ATCC No. 31244), X2282, RR1 (ATCC No. 31343) MRCI; strains of *Bacillus subtilis*; and other enterobacteriaceae such as *Salmonella typhimurium* or *Serratia marcescens*, and various species of *Pseudomonas*. General methods for deriving bacterial strains, such as *E. coli* K12 X1776, useful in the expression of eukaryotic proteins is disclosed by Curtis III in U.S. Patent 4,190,495.

In addition to prokaryotic and eukaryotic microorganisms, expression systems comprising cells derived from multicellular organisms may also be used to produce proteins of the invention. Of particular interest are mammalian expression systems because their post-translational processing machinery is more likely to produce biologically active mammalian proteins. Several DNA tumor viruses have been used as vectors for mammalian hosts. Particularly important are the numerous vectors which comprise SV40 replication, transcription, and/or translation control sequences coupled to bacterial replication control sequences, e.g. the pcD vectors developed by Okayama and Berg, disclosed in Mol. Cell. Biol., Vol. 2, pgs. 161-170 (1982) and Mol. Cell. Biol., Vol. 3, pgs. 280-289 (1983), and improved by Takebe et al., Mol. Cell. Biol., Vol. 8, pgs. 466-472 (1988). Other SV40-based mammalian expression vectors include those disclosed by Kaufman and Sharp, in Mol. Cell. Biol., Vol. 2, pgs. 1304-1319 (1982), and Clark et al., in U.S. patent 4,675,285, both of which are incorporated herein by reference. Monkey cells are usually the preferred hosts for the above vectors. Such vectors containing the SV40 ori sequences and an intact A gene can replicate autonomously in monkey cells (to give higher copy numbers and/or more stable copy numbers than nonautonomously replicating plasmids). Moreover, vectors containing the SV40 ori sequences without an intact A gene can replicate autonomously to high copy numbers (but not stably) in COS7 monkey cells, described by Gluzman, Cell, Vol. 23, pgs. 175-182 (1981) and available from the ATCC (accession no. CRL 1651). The above SV40-based vectors are also capable of transforming other mammalian cells, such as mouse L cells, by integration into the host cell DNA.

Multicellular organisms can also serve as hosts for the production of the polypeptides of the invention, e.g. insect larvae, Maeda et al., Nature, Vol. 315, pgs. 592-594 (1985) and Ann. Rev. Entomol., pgs. 351-372 (1989); and transgenic animals, Jaenisch, Science, Vol. 240, pgs. 1468-1474 (1988).

### I. Assays for Interleukin-10

IL-10s exhibit several biological activities which could form the basis of assays and units. In particular, IL-10s have property of inhibiting the synthesis of at least one cytokine in the group consisting of IFN-γ, lymphotoxin, IL-2, IL-3, and GM-CSF in a population of T helper cells induced to synthesize one or more of these cytokines by exposure to syngeneic antigen-presenting cells (APCs) and antigen. In this activity, the APCs are treated so that they are incapable of replication but, their antigenprocessing machinery remains functional. This is conveniently accomplished by irradiating the APCs, e.g. with about 1500-3000 R (gamma or X-radiation) before mixing with the T cells.

Alternatively, cytokine inhibition may be assayed in primary or, preferably, secondary mixed-lymphocyte reactions (MLR), in which case syngeneic APCs need not be used. MLRs are well known in the art, e.g. Bradley, pgs. 162-166, in Mishell et al., eds. Selected Methods in Cellular Immunology (Freeman, San Francisco, 1980); and Battisto et a., Meth. in Enzymol., Vol. 150, pgs. 83-91 (1987). Briefly, two populations of allogenic lymphoid cells are mixed, one of the populations having been treated prior to mixing to prevent proliferation, e.g. by irradiation. Preferably, the cell populations are prepared at a concentration of about 2 x 10⁶ cells/ml in supplemented medium, e.g. RPMI 1640 with 10% fetal calf serum. For both controls and test cultures, mix 0.5 ml of each population for the assay. For a secondary MLR, the cells remaining after 7 days in the primary MLR are restimulated by freshly prepared, irradiated stimulator cells. The sample suspected of containing IL-10 may be added to the test cultures at the time of mixing, and both controls and test cultures may be assayed for cytokine production from 1 to 3 days after mixing.

Obtaining T cell populations and/or APC populations for IL-10 assays employs techniques well known in the art which are fully described in DiSabato et al., eds., Meth. in Enzymol., Vol. 108 (1984). APCs for the preferred IL-10 assay are peripheral blood monocytes. These are obtained using standard techniques, e.g. as described by Boyum, Meth. in Enzymol., Vol. 108, pgs. 88-102 (1984); Mage, Meth. in Enzymol., Vol. 108, pgs. 118-132 (1984); Litvin et al., Meth. in Enzymol., Vol. 108, pgs. 298-302 (1984); Stevenson, Meth. in Enzymol., Vol. 108, pgs. 242-249 (1989); and Romain et al., Meth. in Enzymol., Vol. 108, pgs. 148-153 (1984), which references are incorporated by reference. Preferably, helper T cells are used in the IL-10 assays, which are obtained by first separating lymphocytes from the peripheral blood then selecting, e.g. by panning or flow cytometry, helper cells using a commercially available anti-CD4 antibody, e.g., OKT4 described in U.S. patent 4,381,295 and available from Ortho Pharmaceutical Corp. The requisite techniques are fully disclosed by Boyum in Scand. J. Clin. Lab. Invest., Vol. 21 (Suppl. 97), pg. 77 (1968) and in Meth. in Enzymol., Vol. 108 (cited above), and by Bram et al., Meth. in Enzymol., Vol. 121, pgs. 737-748 (1986). Generally, PBLs are obtained from fresh blood by Ficoll-Hypaque density gradient centrifugation.

A variety of antigens can be employed in the assay, e.g. Keyhole limpet hemocyanin (KLH), fowl γ-globulin, or the like. More preferably, in place of antigen, helper T cells are stimulated with anti-CD3 monoclonal antibody, e.g. OKT3 disclosed in U.S. patent 4,361,549, in the assay.

Cytokine concentrations in control and test samples are measured by standard biological and/or immunochemical assays. Construction of immunochemical assays for specific cytokines is well known in the art when the purified cytokine is available, e.g. Campbell, Monoclonal Antibody Technology (Elsevier, Amsterdam, 1984); Tijssen, Practice and Theory of Enzyme Immunoassays (Elsevier, Amsterdam, 1985); and U.S. patent 4,486,530 are exemplary of the extensive literature on the subject. ELISA kits for human IL-2, human IL-3, and human GM-CSF are commercially available from Genzyme Corp. (Boston, MA); and an ELISA kit for human IFN-γ is commercially available from Endogen, Inc. (Boston, MA). Polyclonal antibodies specific for human lymphotoxin are available from Genzyme Corp. which can be used in a radioimmunoassay for human lymphotoxin, e.g. Chard, An Introduction to Radioimmunoassay and Related Techniques (Elsevier, Amsterdam, 1982).

Biological assays of the cytokines listed above can also be used to determine IL-10 activity. A biological assay for human lymphotoxin is disclosed by Aggarwal, Meth. in Enzymol., Vol. 116, pgs. 441-447 (1985), and Matthews et al., pgs. 221-225, in Clemens et al., eds., Lymphokines and Interterons: A Practical Approach (IRL Press, Washington, D.C., 1987). Human IL-2 and GM-CSF can be assayed with factor dependent cell lines CTLL-2 and KG-1, available from the ATCC under accession numbers TIB 214 and CCL 246, respectively. Human IL-3 can be assayed by its ability to stimulate the formation of a wide range of hematopoietic cell colonies in soft agar cultures, e.g. as described by Metcalf, The Hemopoietic Colony Stimulating Factors (Elsevier, Amsterdam, 1984). INF-γ can be quantified with anti-viral assays, e.g. Meager, pgs. 129-147, in Clemens et al., eds. (cited above).

Cytokine production can also be determined by mRNA analysis. Cytokine mRNAs can be measured by cytoplasmic dot hybridization as described by White et al., J. Biol. Chem., Vol. 257, pgs. 8569-8572 (1982) and Gillespie et al., U.S. patent 4,483,920. Other approaches include dot blotting using purified RNA, e.g. chapter 6, in Hames et al., eds., Nucleic Acid Hybridization A Practical Approach (IRL Press, Washington, D.C., 1985).

In some cases, samples to be tested for IL-10 activity must be pretreated to remove predetermined cytokines that might interfere with the assay. For example, IL-2 increases the production of IFN-γ in some cells. Thus depending on the helper T cells used in the assay, IL-2 may have to be removed from the sample being tested. Such removals are conveniently accomplished by passing the sample over a standard anti-cytokine affinity column.

For convenience, units of IL-10 activity are defined in terms of IL-10's ability to augment the IL-4-induced proliferation of MC/9 cells, which are described in U.S. patent 4,559,310 and available from the ATCC under accession number CRL 8306. 1 unit/ml is defined as the concentration of IL-10 which gives 50% of maximum stimulation of MC/9 proliferation above the level of IL-4 in the following assay. Prepare duplicate or triplicate dilutions of IL-4 and IL-10 in 50 µl of medium per well in a standard microtiter plate; medium consists of RPMI 1640, 10% fetal calf serum, 50 µM 2-mercaptoethanol, 2 mM glutamine, penicillin (100 U/L) and streptomycin (100 µg/L). Add IL-4, 25 µl/well of 1600 U/ml (400 U/ml final) diluted in medium and incubate overnight, e.g. 20-24 hours. Add ³H-thymidine (e.g. 50 µCi/ml in medium) at 0.5-1.0 µCi/well and again incubate the cells overnight; thereafter harvest the cells and measure the incorporated radioactivity.

### II. Purification and Pharmaceutical Compositions

When polypeptides of the present invention are expressed in soluble form, for example as a secreted product of transformed yeast or mammalian cells, they can be purified according to standard procedures of the art, including steps of ammonium sulfate precipitation, ion exchange chromatography, gel filtration, electrophoresis, affinity chromatography, and/or the like, e.g. "Enzyme Purification and Related Techniques," Methods in Enzymology, 22:233-577 (1977), and Scopes, R., Protein Purification: Principles and Practice (Springer-Verlag, New York, 1982) provides guidance in such purifications. Likewise, when polypeptides of the invention are expressed in insoluble form, for example as aggregates, inclusion bodies, or the like, they can be purified by standard procedures in the art, including separating the inclusion bodies from disrupted host cells by centrifugation, solubilizing the inclusion bodies with chaotropic agents and reducing agents, diluting the solubilized mixture, and lowering the concentration of chaotropic agent and reducing agent so that the polypeptide takes on a biologically active conformation. The latter procedures are disclosed in the following references, Winkler et al., Biochemistry, 25:4041-4045 (1986); Winkler et al., Biotechnology, 3: 992-998 (1985); Koths et al., U.S. patent 4,569,790; and European patent applications 86306917.5 and 86306353.3.

As used herein "effective amount" means an amount sufficient to reduce or prevent tumor growth. The effective amount for a particular patient may vary depending on such factors as the state and type of the neoplastic disease being treated, the overall health of the patient, method of administration, the severity of side-effects, and the like. Generally, IL-10 is administered as a pharmaceutical composition comprising an effective amount of IL-10 and a pharmaceutical carrier. A pharmaceutical carrier can be any compatible, non-toxic substance suitable for delivering the compositions of the invention to a patient. Generally, compositions useful for parenteral administration of such drugs are well known, e.g. Remington's Pharmaceutical Science, 15th Ed. (Mack Publishing Company, Easton, PA 1980). Alternatively, compositions of the invention may be introduced into a patient's body by implantable or injectable drug delivery system: e.g. Urquhart et al., Ann. Rev. Pharmacol. Toxicol., Vol. 24, pgs. 199-236 (1984); Lewis, ed. Controlled Release of Pesticides and Pharmaceuticals (Plenum Press, New York, 1981); U.S. patent 3,773,919; U.S. patent 3,270,960.

When administered parenterally, the IL-10 is formulated in a unit dosage injectable form (solution, suspension, emulsion) in association with a pharmaceutical carrier. Examples of such carriers are normal saline, Ringers solution, dextrose solution, and Hank's solution. Nonaqueous carriers such as fixed oils and ethyl oleate may also be used. A preferred carrier is 5% dextrose/saline. The carrier may contain minor amounts of additives such as substances that enhance isotonicity and chemical stability, e.g., buffers and preservatives. The IL-10 is preferably formulated in purified form substantially free of aggregates and other proteins at a concentration in the range of about 5 to 20 µg/ml. Preferably, IL-10 is administered by continuous infusion so that an amount in the range of about 50-800 µg is delivered per day (i.e. about 1-16 µg/kg/day). The daily infusion rate may be varied based on monitoring of side effects and blood cell counts.

### EXAMPLES

The following examples serve to illustrate the present invention. The selected vectors and hosts, the concentration of reagents, the temperatures, and the values of other variables are only to exemplify application of the present invention and are not to be considered limitations thereof.

### Example 1. Expression of human CSIF in a bacterial host

A synthetic human CSIF gene is assembled from a plurality of chemically synthesized double-stranded DNA fragments to form an expression vector designated TAC-RBS-hCSIF. Cloning and expression are carried out in a standard bacterial system, for example *E. coli* K-12 strain JM101, JM103, or the like, described by Viera and Messing, in Gene, Vol. 19, pgs. 259-268 (1982). Digestions with restriction endonucleases and reactions with ligases are performed using standard protocols, e.g. Maniatis et al., Molecular Cloning: A Laboratory Manual (Cold Spring Harbor Laboratory, New York, 1982).

The alkaline method (Maniatis et al., cited above) is used for small-scale plasmid preparations. For large-scale preparations a modification of the alkaline method is used in which an equal volume of isopropanol is used to precipitate nucleic acids from the cleared lysate. Precipitation with cold 2.5 M ammonium acetate is used to remove RNA prior to equilibrium density centrifugation with cesium chloride and detection with ethidium bromide.

For filter hybridizations Whatman 540 filter circles are used to lift colonies which are then lysed and fixed by treatment (for 2 minutes each) with 0.5M NaOH, 1 .5M NaCl and then with 1 M Tris.HCl pH8.0, 1.5M NaCl, and then by heating at 80°C (30 min). Hybridizations are in 6xSSPE, 20% formamide, 0.1% sodium dodecylsulphate (SDS), 100 mg/ml *E. coli* tRNA, 100 mg/ml Coomassie Brilliant Blue G-250 (Bio-Rad) at 42°C for 6 hrs using ³²P-labelled (kinased) synthetic DNAs. (20xSSPE is prepared by dissolving 174 g of NaCl, 27.6 g of NaH₂PO₄.9H₂O, and 7.4 g of EDTA in 800 ml of H₂O. pH is adjusted to 7.4 with NaOH, volume is adjusted to 1 liter, and the sample is sterilized by autoclaving.) Filters are washed twice (15 min, room temperature) with 1xSSPE, 0.1% SDS. After autoradiography (Fuji RX film), positive colonies are located by aligning the regrown colonies with the blue-stained colonies on the filters. DNA is sequenced by the dideoxy method, Sanger et al. Proc. Natl. Acad. Sci., Vol. 74, pg. 5463 (1977). Templates for the dideoxy reactions are either single-stranded DNAs of relevant regions recloned into M13mp vectors (e.g. Messing et al. Nucleic Acids Res., Vol. 9, pg. 309 (1981)), or double-stranded DNA prepared by the minialkaline method and denatured with 0.2M NaOH (5 min, room temperature) and precipitated from 0.2M NaOH, 1.43M ammonium acetate by the addition of 2 volumes of ethanol. DNA can be synthesized by phosphoramidite chemistry using Applied Biosystems 380A synthesizers; synthesis, deprotection, cleavage and purification (7M urea PAGE, elution, DEAE-cellulose chromotography) are done as described in the 380A synthesizer manual.

Complementary strands of synthetic DNAs to be cloned (400 ng each) are mixed and phosphorylated with polynucleotide kinase in a reaction volume of 50 ml. This DNA is ligated in a volume of 50 ml at room temperature for 4 to 12 hours with 1 mg of vector DNA digested with appropriate restriction enzyme. Conditions for phosphorylation, digestion with restriction enzymes, polymerase reactions, and ligation have been described (Maniatis et al., cited above). Colonies are scored for lacZ+ (when desired) by plating on L agar supplemented with ampicillin, isopropyl-1-thio-beta-D-galactoside (IPTG) (0.4 mM) and 5-bromo-4-chloro-3-indolyl-beta-D-galactopyranoside (x-gal) (40 mg/ml).

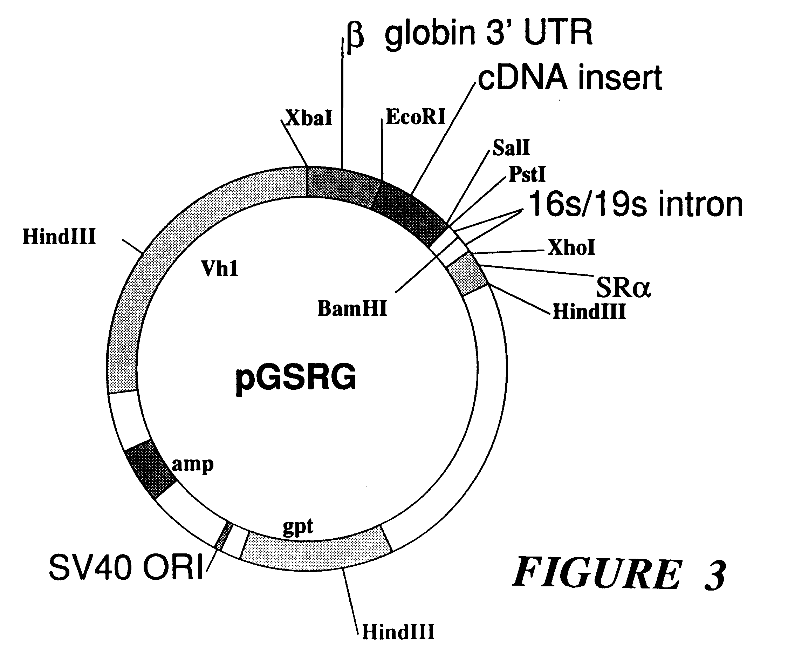

The TAC-RBS vector is constructed by filling-in with DNA polymerase the single *Bam* HI site of the tacP-bearing plasmid pDR540 (Pharmacia). This is then ligated to unphosphorylated synthetic oligonucleotides (Pharmacia) which form a double-stranded fragment encoding a consensus ribosome binding site as given in SEQ. ID. NO. 5 herein and designated RBS. After ligation, the mixture is phosphorylated and religated with the *Sst* I linker ATGAGCTCAT. This complex was then cleaved with *Sst* I and *Eco* RI, and the 173 base pair (bp) fragment isolated by polyacrylamide gel electrophoresis (PAGE) and cloned into *Eco* RI-*Sst* I-restricted pUC19 (Pharmacia) (as described below). The sequence of the RBS-ATG-polylinker regions of the final construction (called TAC-RBS) is shown in Figure 3.

The synthetic IL-10 gene is assembled into a pUC19 plasmid in eight steps. At each step, inserts free of deletions and/or inserts can be detected after cloning by maintaining the lacZ(α) gene of pUC19 in frame with the ATG start codon inserted in step 1. Clones containing deletion and/or insertion changes can be filtered out by scoring for blue colonies on L-ampicillin plates containing x-gal and IPTG. Alternatively, at each step sequences of inserts can be readily confirmed using a universal sequencing primer on small-scale plasmid DNA preparations, e.g. available from Boehringer Mannheim.

In step 1 the TAC-RBS vector is digested with *Sst*I, treated with T4 DNA polymerase (whose 3'-exonuclease activity digests the 3'-protruding strands of the *Sst*I cuts to form blunt end fragments) and, after deactivation of T4 DNA polymerase, treated with *Eco* RI to form a 173 bp fragment containing the TAC-RBS region and having a blunt end at the ATG start codon and the *Eco* RI cut at the opposite end. Finally, the 173 bp TAC-RBS fragment is isolated.

In step 2 the isolated TAC-RBS fragment of step 1 is mixed with *Eco* RI/*Kpn* I-digested plasmid pUC19 and synthetic fragment 1A/B shown in SEQ. ID. NO. 6 herein which, as shown below, has a blunt end at its upstream terminus and a staggered end corresponding to a *Kpn* I cut at its downstream terminus. This *Kpn* I end is adjacent to and downstream of a *Bst* EII site. The fragments are ligated to form the pUC19 of step 2.

In step 3 synthetic fragment 2A/B shown in SEQ. ID. NO. 7 herein and 3A/B shown in SEQ. ID. NO. 8 herein are mixed with *Bst* EII/*Sma*I-digested pUC19 of step 2 (after amplification and purification) and ligated to form pUC19 of step 3. Note that the downstream terminus of fragment 3A/B contains extra bases which form the *Sma*I blunt-end. These extra bases are cleaved in step 4. Also fragments 2A/B and 3A/B have complementary 9-residue single-stranded ends which anneal upon admixture, leaving the upstream *Bst*EII cut of 2A/B and the downstream blunt-end of 3A/B to ligate to the pUC19.

In step 4, the pUC19 of step 3 is digested with *Afl* II/*Xba* I, amplified, purified, repurified, mixed with synthetic fragment 4A/B shown in SEQ. ID. NO. 9 herein, and ligated to form pUC19 of step 4.

In step 5 pUC19 of step 4 is digested with *Xba*I/*Sal*I, amplified and purified, and mixed with synthetic fragment 5A/B shown in SEQ. ID. NO. 10 herein and ligated to form the pUC19 of step 5. Note that the *Sal*I-staggered end of fragment 5A/B is eliminated by digestion with *Hpa*I in step 6.

In step 6 pUC19 of step 5 is digested with *Hpa*I/Pst I, amplified and purified, and mixed with synthetic fragment 6A/B shown in SEQ. ID. NO. 11 herein and ligated to form the pUC19 of step 6.

In step 7 pUC19 of step 6 is digested with *Cla*I/*Sph*I, amplified and purified, and mixed with synthetic fragment 7A/B shown in SEQ. ID. NO. 12 herein and ligated to form the pUC19 of step 7.

In step 8 pUC19 of step 7 is digested with *Mlu*I/*Hin*dIII, amplified and purified, and mixed with synthetic fragments 8A/B shown in SEQ. ID. NO. 13 herein and 9A/B shown in SEQ. ID. NO. 14 herein and ligated to form the final construction, which is then inserted into *E. coli* K-12 strain JM101, e.g. available from the ATCC under accession number 33876, by standard techniques. After cultivation, protein is extracted from the JM101 cells and dilutions of the extracts are tested for biological activity.

### Example 2. Expression of vIL-10 in COS 7 Monkey cells

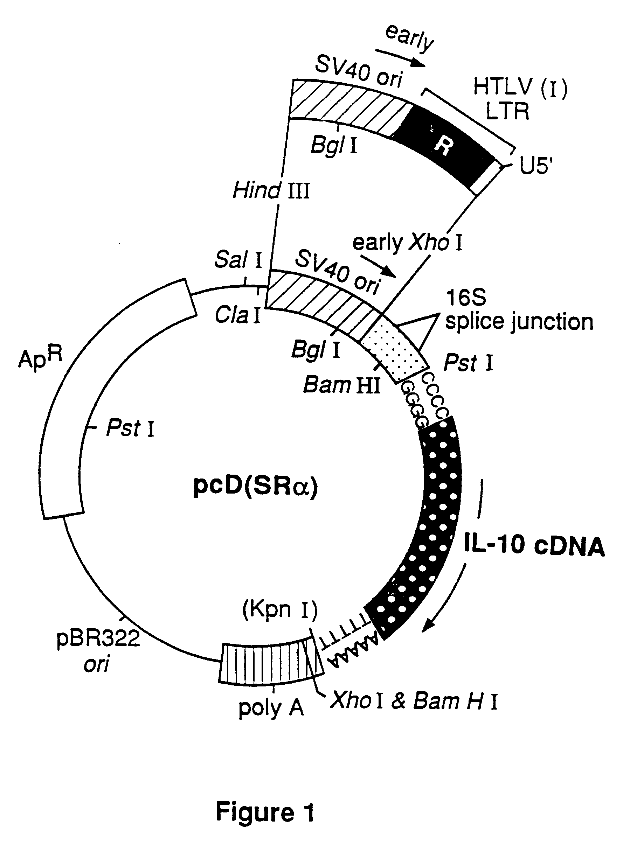

A gene encoding the open reading frame for vIL-10 was amplified by polymerase chain reaction using primers that allowed later insertion of the amplified fragment into an *Eco* RI-digested pcD(SRα) vector (Figure 1). The coding strand of the inserted fragment is shown in SEQ. ID. NO. 15 herein.

Clones carrying the insert in the proper orientation were identified by expression of vIL-10 and/or the electrophoretic pattern of restriction digests. One such vector carrying the vIL-10 gene was designated pBCRF1(SRα) and was deposited with the ATCC under accession number 68193. pBCRF1(SRα) was amplified in *E. coli* MC1061, isolated by standard techniques, and used to transfect COS 7 monkey cells as follows: One day prior to transfection, approximately 1.5 x 10⁶ COS 7 monkey cells were seeded onto individual 100 mm plates in Dulbecco's modified Eagle medium (DME) containing 5% fetal calf serum (FCS) and 2 mM glutamine. To perform the transfection, COS 7 cells were removed from the dishes by incubation with trypsin, washed twice in serum-free DME, and suspended to 10⁷ cells/ml in serum-free DME. A 0.75 ml aliquot was mixed with 20 µg DNA and transferred to a sterile 0.4 cm electroporation cuvette. After 10 minutes, the cells were pulsed at 200 volts, 960 µF in a BioRad Gene Pulser unit. After another 10 minutes, the cells were removed from the cuvette and added to 20 ml of DME containing 5% FCS, 2mM glutamine, penicillin, streptomycin, and gentamycin. The mixture was aliquoted to four 100 mm tissue culture dishes. After 12-24 hours at 37°C, 5% CO₂, the medium was replaced with similar medium containing only 1% FCS and the incubation was continued for an additional 72 hours at 37°C, 5% CO₂, after which the medium was collected and assayed for its ability to inhibit IFN-γ synthesis.

10 ml aliquots of freshly isolated PBLs (about 2x10⁶ cells/ml) were incubated at 37°C with PHA (100 ng/ml) in medium consisting of (i) 90% DME supplemented with 5% FCS and 2 mM glutamine, and (ii) 10% supernatant from COS 7 cells previously transfected with pBCRF1(SRα). After 24 hours the cells and supernatants were harvested to assay for the presence of either IFN-γ mRNA or IFN-γ protein, respectively. Controls were treated identically, except that the 10% supernatant was from COS 7 cultures previously transfected with a plasmid carrying an unrelated cDNA insert. The vIL-10-treated samples exhibited about a 50% inhibition of IFN-γ synthesis relative to the controls.

### Example 3. Expression of vIL-10 in Escherichia coli

A gene encoding the mature vIL-10 shown in SEQ. ID. NO. 4 herein may be expressed in *E.coli*.

The cDNA insert of pBCRF1(SRα) is recloned into an M13 plasmid where it is altered twice by site-directed mutagenesis: first to form a *Cla*I site at the 5'-end of the coding region for the mature vIL-10 polypeptide, and second to form a *Bam*HI site at the 3'-end of the coding region for the mature vIL-10 polypeptide. The mutated sequence is then readily inserted into the TRPC11 expression vector described below.

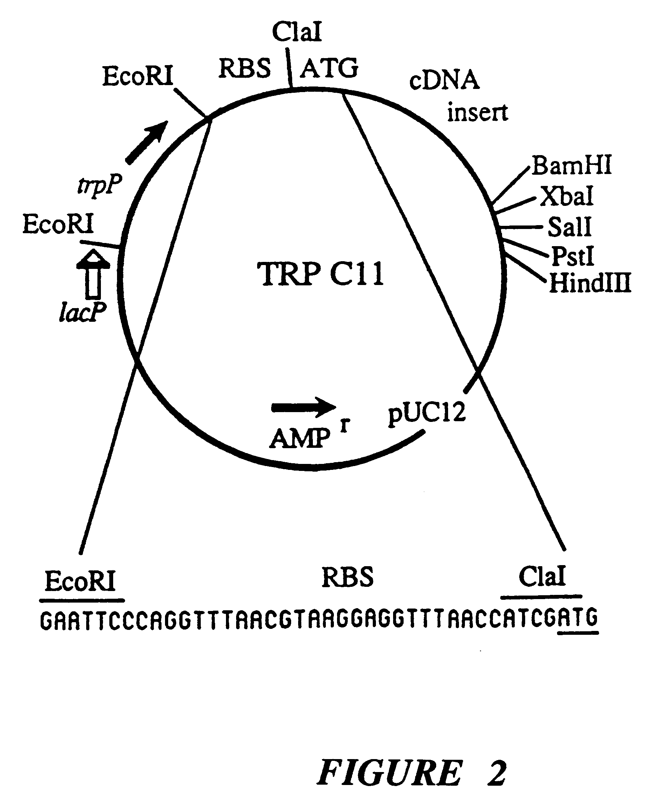

The TRPC11 vector was constructed by ligating a synthetic consensus RBS fragment to *Cla*I linkers (ATGCAT) and by cloning the resulting fragments into *Cla*I-restricted pMT11hc (which had been previously modified to contain the *Cla*I site). pMT11hc is a small (2.3 kilobase) high copy, AMP^{R}, TET^{S} derivative of pBR322 that bears the πVX plasmid *Eco* RI-*Hin*dIII polylinker region. (πVX is described by Maniatis et al., Molecular Cloning: A Laboratory Manual, Cold Spring Harbor Laboratory, 1982.) This was modified to contain the *Cla*I site by restricting pMT11hc with *Eco* RI and *Bam* HI, filling in the resulting sticky ends and ligating with *Cla*I linker (CATCGATG), thereby restoring the *Eco* RI and *Bam* HI sites and replacing the *Sma*I site with a *Cla*I site. One transformant from the TRPC11 construction had a tandem RBS sequence flanked by *Cla*I sites. One of the *Cla*I sites and part of the second copy of the RBS sequence were removed by digesting this plasmid with *Pst*I, treating with *Bal*31 nuclease, restricting with *Eco* RI and treating with T4 DNA polymerase in the presence of all four deoxynucleotide triphosphates. The resulting 30-40 bp fragments were recovered by PAGE and cloned into *Sma*I-restricted pUC12. A 248 bp *E*. *coli* trpP-bearing *Eco* RI fragment derived from pKC101 (described by Nichols et al. in Methods in Enzymology, Vol. 101, pg. 155 (Academic Press, N.Y. 1983)) was then cloned into the *Eco* RI site to complete the TRPC11 construction, which is illustrated in Figure 2. TRPC11 is employed as a vector for vIL-10 by first digesting it with *Cla*I and *Bam* HI, purifying it, and then mixing it in a standard ligation solution with the *ClaI*-*Bam*HI-fragment of the M13 containing the nucleotide sequence coding for the mature BCRF1. The insert-containing TRPC11, referred to as TRPC11-BCRF1, is propagated in *E. coli* K12 strain JM101, e.g. available from the ATCC under accession number 33876.

### Example 4. Suppression of tumors in mice by IL-10

The effect of IL-10 on tumor growth was tested in an assay similar to that described by Tepper et al., Cell, Vol., 57, pgs. 503-512 (1989). Briefly, the assay as applied here involves separately injecting mice with two groups of cells from a syngenic tumor cell line, one group being stably transfected with an IL-10-expressing plasmid, and the other being a non-transfected control. The incidence of tumor formation was then compared in mice separately injected with cells of the two groups.

In all experiments BALB/c mice were injected with either transfected or non-transfected NS1 myeloma tumor cells, available from the ATCC under accession number TIB 18. NS1 cells were transfected by electroporation with plasmid pGSRG (illustrated in Fig. 3) carrying the open reading frame (ORF) of mouse IL-10, viral IL-10, or human IL-10 inserted into the *Xho* I restriction site. pGSRG contains a marker for determining stably transfected NS1 cells and is a derivative of pSVDtβ described by Schnee et al., Proc. Natl. Acad. Sci., Vol. 84, pgs. 6904-6908 (1987). The IL-10 inserts for pGSRG were obtained as follows. PCR primers containing *Eco* RI linker sequences were prepared for each of pH5C, pBCRF1, and pcD(SRα)-F115 so that the ORFs of the respective cDNA inserts could be amplified. The amplified ORFs were then recloned into *Eco* RI-digested pcD(SRα) vectors and separately expressed to select vectors containing the ORFs in the correct orientation. Finally, the *Xho* I fragments of the pcD(SRα)s were recloned into respective *Xho* I-digested pGSRG plasmids.

The NS1 cells were transfected with the pGSRGs as follows. Cells (2-3 x 10⁶) from a half-confluent 100 mm diameter petri dish were centrifuged, washed once in 10 ml of sterile phosphate buffered saline (PBS), centrifuged, and resuspended in 0.5 ml PBS. 20-100 µg of plasmid DNA were suspended in 1 ml PBS. The cells and DNA were mixed, placed in cuevette on ice, and electrically shocked in a Bio-Rad Gene Pulser set to 960 microFarad capacitance and 200-300 volts. After 10 min. on ice the cells were then plated onto a standard 96-well microtiter plate in 100 µl volumes. 48-78 hours later, 100 µl of mycophenolic acid selection medium (0.5 µg/ml mycophenolic acid, 100 µg/ml xanthine, 15 ug/ml hypoxanthine, 12-15% fetal calf serum, 600 µg/ml glutamine in DME High Glucose) was added to each well.

In a first experiment, 4, 3, 4, and 5 mice respectively were each injected intraperitoneally with 5 x 10⁶ non-transformed cells (control), pGSRG-mIL 10-transformed cells, pGSRG-vIL 10-transformed cells, and pGSRG-hIL 10-transformed cells. After 4 weeks, 3 out of 4 of the control mice had developed visable tumors; none of the experimental mice developed tumors after 4 weeks.

In a second experiment, 16 mice were intraperitoneally injected with 5 x 10⁶ non-transformed cells and 15 mice were intraperitoneally injected with 5 x 10⁶ pGSRG-mIL 10-transformed cells. After two weeks, 8 of the 16 control mice were re-injected with 5 x 10⁶ non-transformed cells, and 7 of the 15 experimental mice were re-injected with 5 x 10⁶ pGSRG-mIL 10-transformed cells. 4 weeks after the initial injections the mice were examined for evidence of tumor development. 16 out of 16 of the control mice had developed visable tumors, and none of the experimental mice had developed any signs of tumors.

The descriptions of the foregoing embodiments of the invention have been presented for purpose of illustration and description. They are not intended to be exhaustive or to limit the invention to the precise forms disclosed. The embodiments were chosen and described in order to best explain the principles of the invention to thereby enable others skilled in the art to best utilize the invention in various embodiments and with various modifications as are suited to the particular use contemplated. It is intended that the scope of the invention should be defined by the claims appended hereto.

Applicants have deposited separate cultures of *E. coli* MC1061 carrying pH5C, pH15C, and pBCRF1(SRα) with the American Type Culture Collection, Rockville, MD, USA (ATCC), under accession numbers 68191, 68192, and 68193, respectively. These deposits were made under conditions as provided under ATCC's agreement for Culture Deposit for Patent Purposes, which assures that the deposit will be made available to the US Commissioner of Patents and Trademarks pursuant to 35 USC 122 and 37 CFR 1.14, and will be made available to the public upon issue of a U.S. patent, which requires that the deposit be maintained. Availability of the deposited strain is not to be construed as a license to practice the invention in contravention of the rights granted under the authority of any government in accordance with its patent laws.

SEQUENCE LISTING

SEQ ID NO: 1
SEQUENCE TYPE: Amino acid
SEQUENCE LENGTH: 178 amino acid residues
STRANDEDNESS: single
TOPOLOGY: linear
MOLECULE TYPE: Protein
ORIGINAL SOURCE ORGANISM: Human
PROPERTIES: Human IL-10 (human cytokine synthesis
inhibitory factor, human CSIF)

Met His Ser Ser Ala Leu Leu Cys Cys Leu Val Leu Leu Thr Gly
                5                   10                  15
Val Arg Ala Ser Pro Gly Gln Gly Thr Gln Ser Glu Asn Ser Cys
                20                  25                  30
Thr His Phe Pro Gly Asn Leu Pro Asn Met Leu Arg Asp Leu Arg
                35                  40                  45
Asp Ala Phe Ser Arg Val Lys Thr Phe Phe Gln Met Lys Asp Gln
                50                  55                  60
Leu Asp Asn Leu Leu Leu Lys Glu Ser Leu Leu Glu Asp Phe Lys
                65                  70                  75
Gly Tyr Leu Gly Cys Gln Ala Leu Ser Glu Met Ile Gln Phe Tyr
                80                  85                  90
Leu Glu Glu Val Met Pro Gln Ala Glu Asn Gln Asp Pro Asp Ile
                95                  100                 105
Lys Ala His Val Asn Ser Leu Gly Glu Asn Leu Lys Thr Leu Arg
                110                 115                 120
Leu Arg Leu Arg Arg Cys His Arg Phe Leu Pro Cys Glu Asn Lys
                125                 130                 135
Ser Lys Ala Val Glu Gln Val Lys Asn Ala Phe Asn Lys Leu Gln
                140                 145                 150
Glu Lys Gly Ile Tyr Lys Ala Met Ser Glu Phe Asp Ile Phe Ile
                155                 160                 165
Asn Tyr Ile Glu Ala Tyr Met Thr Met Lys Ile Arg Asn
                170                 175

SEQ ID NO: 2
SEQUENCE TYPE: Amino acid
SEQUENCE LENGTH: 170 amino acid residues

STRANDEDNESS: single
TOPOLOGY: linear
MOLECULE TYPE: Protein
PROPERTIES: Viral IL-10 (BCRF1)

Met Glu Arg Arg Leu Val Val Thr Leu Gln Cys Leu Val Leu Leu
                5                   10                  15
Tyr Leu Ala Pro Glu Cys Gly Gly Thr Asp Gln Cys Asp Asn Phe
                20                  25                  30
Pro Gln Met Leu Arg Asp Leu Arg Asp Ala Phe Ser Arg Val Lys
                35                  40                  45
Thr Phe Phe Gln Thr Lys Asp Glu Val Asp Asn Leu Leu Leu Lys
                50                  55                  60
Glu Ser Leu Leu Glu Asp Phe Lys Gly Tyr Leu Gly Cys Gln Ala
                65                  70                  75
Leu Ser Glu Met Ile Gln Phe Tyr Leu Glu Glu Val Met Pro Gln
                80                  85                  90
Ala Glu Asn Gln Asp Pro Glu Ala Lys Asp His Val Asn Ser Leu
                95                  100                 105
Gly Glu Asn Leu Lys Thr Leu Arg Leu Arg Leu Arg Arg Cys His
                110                 115                 120
Arg Phe Leu Pro Cys Glu Asn Lys Ser Lys Ala Val Glu Gln Ile
                125                 130                 135
Lys Asn Ala Phe Asn Lys Leu Gln Glu Lys Gly Ile Tyr Lys Ala
                140                 145                 150
Met Ser Glu Phe Asp Ile Phe Ile Asn Tyr Ile Glu Ala Tyr Met
                155                 160                 165
Thr Ile Lys Ala Arg
                170

SEQ ID NO: 3
SEQUENCE TYPE: Amino acid
SEQUENCE LENGTH: 160 amino acid residues
STRANDEDNESS: single
TOPOLOGY: linear
MOLECULE TYPE: Protein
ORIGINAL SOURCE ORGANISM: Human
PROPERTIES: Mature human IL-10 (mature human CSIF)

Ser Pro Gly Gln Gly Thr Gln Ser Glu Asn Ser Cys Thr His Phe
                5                   10                  15
Pro Gly Asn Leu Pro Asn Met Leu Arg Asp Leu Arg Asp Ala Phe
                20                  25                  30
Ser Arg Val Lys Thr Phe Phe Gln Met Lys Asp Gln Leu Asp Asn
                35                  40                  45
Leu Leu Leu Lys Glu Ser Leu Leu Glu Asp Phe Lys Gly Tyr Leu
                50                  55                  60
Gly Cys Gln Ala Leu Ser Glu Met Ile Gln Phe Tyr Leu Glu Glu
                65                  70                  75
Val Met Pro Gln Ala Glu Asn Gln Asp Pro Asp Ile Lys Ala His
                80                  85                  90
Val Asn Ser Leu Gly Glu Asn Leu Lys Thr Leu Arg Leu Arg Leu
                95                  100                 105
Arg Arg Cys His Arg Phe Leu Pro Cys Glu Asn Lys Ser Lys Ala
                110                 115                 120
Val Glu Gln Val Lys Asn Ala Phe Asn Lys Leu Gln Glu Lys Gly
                125                 130                 135
Ile Tyr Lys Ala Met Ser Glu Phe Asp Ile Phe Ile Asn Tyr Ile
                140                 145                 150
Glu Ala Tyr Met Thr Met Lys Ile Arg Asn
                155                 160

SEQ ID NO:  4
SEQUENCE TYPE:  Amino acid
SEQUENCE LENGTH:  147 amino acid residues
STRANDEDNESS:  single
TOPOLOGY:  linear
MOLECULE TYPE:  Protein
PROPERTIES:  Viral IL-10 (BCRF1)

Thr Asp Gln Cys Asp Asn Phe Pro Gln Met Leu Arg Asp Leu Arg
                5                   10                  15
Asp Ala Phe Ser Arg Val Lys Thr Phe Phe Gln Thr Lys Asp Glu
                20                  25                  30
Val Asp Asn Leu Leu Leu Lys Glu Ser Leu Leu Glu Asp Phe Lys
                35                  40                  45
Gly Tyr Leu Gly Cys Gln Ala Leu Ser Glu Met Ile Gln Phe Tyr
                50                  55                  60

Leu Glu Glu Val Met Pro Gln Ala Glu Asn Gln Asp Pro Glu Ala
                65                  70                  75
Lys Asp His Val Asn Ser Leu Gly Glu Asn Leu Lys Thr Leu Arg
                80                  85                  90
Leu Arg Leu Arg Arg Cys His Arg Phe Leu Pro Cys Glu Asn Lys
                95                  100                 105
Ser Lys Ala Val Glu Gln Ile Lys Asn Ala Phe Asn Lys Leu Gln
                110                 115                 120
Glu Lys Gly Ile Tyr Lys Ala Met Ser Glu Phe Asp Ile Phe Ile
                125                 130                 135
Asn Tyr Ile Glu Ala Tyr Met Thr Ile Lys Ala Arg
                140                 145

SEQ ID NO: 5
SEQUENCE TYPE: Nucleotide
SEQUENCE LENGTH: 15 bases
STRANDEDNESS: single
TOPOLOGY: linear
MOLECULE TYPE: DNA
IMMEDIATE EXPERIMENTAL SOURCE: Synthetic (commercial,
     Pharmacia)
FEATURES: Double-stranded fragment encoding a consensus
     ribosome binding site.

GTAAGGAGGT TTAAC                                        15

SEQ ID NO: 6
SEQUENCE TYPE: Nucleotide
SEQUENCE LENGTH: 56 base pairs with 4-base sticky end
STRANDEDNESS: double
TOPOLOGY: linear
MOLECULE TYPE: DNA
IMMEDIATE EXPERIMENTAL SOURCE: synthetic
FEATURES: Lower case letters indicate that a base differs
     from that of the native sequence at the same site
PROPERTIES: Fragment 1A/B of synthetic CSIF gene

AGCCCAGGCC AGGGCACCCA GTCTGAGAAC AGCTGCACCC ACTTCCCAGG 50
TCGGGTCCGG TCCCGTGGGT CAGACTCTTG TCGACGTGGG TGAAGGGTCC

tAACCggtac 60
aTTGGc

SEQ ID NO: 7

SEQUENCE TYPE: Nucleotide

SEQUENCE LENGTH: 48 base pairs with sticky ends of 5 and
9 bases

STRANDEDNESS: double

TOPOLOGY: linear

MOLECULE TYPE: DNA

IMMEDIATE EXPERIMENTAL SOURCE: synthetic

FEATURES: Lower case letters indicate that a base differs
from that of the native sequence at the same site

PROPERTIES: Fragment 2A/B of synthetic CSIF gene

GtAACCTGCC TAACATGCTT CGAGATCTCC GAGATGCCTT CAGCAGAGTG 50
GACGG ATTGTACGAA GCTCTAGAGG CTCTACGGAA GTCGTCTCAC

AAGACTTTCT TT 62
TTC

SEQ ID NO: 8

SEQUENCE TYPE: Nucleotide

SEQUENCE LENGTH: 35 base pairs with sticky end of 9 bases

STRANDEDNESS: double

TOPOLOGY: linear

MOLECULE TYPE: DNA

IMMEDIATE EXPERIMENTAL SOURCE: synthetic

FEATURES: Lower case letters indicate that a base differs
from that of the native sequence at the same site

PROPERTIES: Fragment 3A/B of synthetic CSIF gene

C AAATGAAGGA TCAGCTGGAC AACTTGTTcT tAAG 35
TGAAAGAAAG TTTACTTCCT AGTCGACCTG TTGAACAAgA aTTC

SEQ ID NO: 9
SEQUENCE TYPE: Nucleotide
SEQUENCE LENGTH: 69 base pairs with 4-base sticky end
STRANDEDNESS: double
TOPOLOGY: linear
MOLECULE TYPE: DNA
IMMEDIATE EXPERIMENTAL SOURCE: synthetic
FEATURES: Lower case letters indicate that a base differs
from
that of the native sequence at the same site
PROPERTIES: Fragment 4A/B of synthetic CSIF gene

GAGTCCTTGC TGGAGGACTT TAAGGGTTAC CTGGGTTGCC AAGCCTTGTC 50
CTCAGGAACG ACCTCCTGAA ATTCCCAATG GACCCAACGG TTCGGAACAG
TGAGATGATC CAGTTTTAt 69
ACTCTACTAG GTCAAAATaG AtC

SEQ ID NO: 10
SEQUENCE TYPE: Nucleotide
SEQUENCE LENGTH: 61 base pairs with 4-base sticky end
STRANDEDNESS: double
TOPOLOGY: linear
MOLECULE TYPE: DNA
IMMEDIATE EXPERIMENTAL SOURCE: synthetic
FEATURES: Lower case letters indicate that a base differs
from that of the native sequence at the same site
PROPERTIES: Fragment 5A/B of synthetic CSIF gene

CTaGAGGAGG TGATGCCCCA AGCTGAGAAC CAAGACCCAG ACATCAAGGC 50
GAtCTCCTCC ACTACGGGGT TCGACTCTTG GTTCTGGGTC TGTAGTTCCG
GCATGTtAAC g 61
CGTACAaTTG cagct

SEQ ID NO: 11
SEQUENCE TYPE: Nucleotide
SEQUENCE LENGTH: 61 base pairs with 4-base sticky end

STRANDEDNESS:  double
TOPOLOGY:  linear
MOLECULE TYPE:  DNA
IMMEDIATE EXPERIMENTAL SOURCE:  synthetic
FEATURES:  Lower case letters indicate that a base differs
     from that of the native sequence at the same site
PROPERTIES:  Fragment 6A/B of synthetic CSIF gene

AACTCCCTGG GGGAGAACCT GAAGACCCTC AGGCTGAGGC TACGGCGCTG    50
TTGAGGGACC CCCTCTTGGA CTTCTGGGAG TCCGACTCCG ATGCCGCGAC
TCATCGATct gca                                            63
AGTAGCTAg

SEQ ID NO:  12
SEQUENCE TYPE:  Nucleotide
SEQUENCE LENGTH:
STRANDEDNESS:  double
TOPOLOGY:  linear
MOLECULE TYPE:  DNA
IMMEDIATE EXPERIMENTAL SOURCE:  synthetic
FEATURES:  Lower case letters indicate that a base differs
     from that of the native sequence at the same site
PROPERTIES:  Fragment 7A/B of synthetic CSIF gene

CGATTTCTTC CCTGTCAAAA CAAGAGCAAG GCCGTGGAGC AGGTGAAGAA    50
  TAAAGAAG GGACAGTTTT GTTCTCGTTC CGGCACCTCG TCCACTTCTT
cGCgTgcatg                                                60
gCGcAc

SEQ ID NO:  13
SEQUENCE TYPE:  Nucleotide
SEQUENCE LENGTH:  45 base pairs and sticky ends of 4 and 9
     bases
STRANDEDNESS:  double
TOPOLOGY:  linear
MOLECULE TYPE:  DNA
IMMEDIATE EXPERIMENTAL SOURCE:  synthetic

FEATURES: Lower case letters indicate that a base differs
from that of the native sequence at the same site
PROPERTIES: Fragment 8A/B of synthetic CSIF gene

CGCGTTTAAT AATAAGCTCC AAGACAAAGG CATCTACAAA GCCATGAGTG 50
AAATTA TTATTCGAGG TTCTGTTTCC GTAGATGTTT CGGTACTCA
AGTTTGAC 58

SEQ ID NO: 14
SEQUENCE TYPE: Nucleotide
SEQUENCE LENGTH: 51 base pairs with sticky ends of 9 and 4
bases
STRANDEDNESS: double
TOPOLOGY: linear
MOLECULE TYPE: DNA
IMMEDIATE EXPERIMENTAL SOURCE: synthetic
FEATURES: Lower case letters indicate that a base differs
from that of the native sequence at the same site
PROPERTIES: Fragment 9A/B of synthetic CSIF gene

A TCTTCATCAA CTACATAGAA GCCTACATGA CAATGAAGAT 50
CTCAAACTGT AGAAGTAGTT GATGTATCTT CGGATGTACT GTTACTTCTA
ACGAAACTGA 60
TGCTTTGACT tcga

SEQ ID NO: 15
SEQUENCE TYPE: Nucleotide
SEQUENCE LENGTH: 499 bases
STRANDEDNESS: single
TOPOLOGY: linear
MOLECULE TYPE: DNA
PROPERTIES: Encodes viral IL-10

AATTC ATG GAG CGA AGG TTA GTG GTC ACT CTG CAG TGC CTG GTG 44
CTG CTT TAC CTG GCA CCT GAG TGT GGA GGT ACA GAC CAA TGT 86
GAC AAT TTT CCC CAA ATG TTG AGG GAC CTA AGA GAT GCC TTC 128
AGT CGT GTT AAA ACC TTT TTC CAG ACA AAG GAC GAG GTA GAT 170

AAC CTT TTG CTC AAG GAG TCT CTG CTA GAG GAC TTT AAG GGC 212
TAC CTT GGA TGC CAG GCC CTG TCA GAA ATG ATC CAA TTC TAC 254
CTG GAG GAA GTC ATG CCA CAG GCT GAA AAC CAG GAC CCG GAG 296
GCT AAG GAC CAT GTC AAT TCT TTG GGT GAA AAT CTA AAG ACC 338
CTA CGG CTC CGC CTG CGC AGG TGC CAC AGG TTC CTG CCG TGT 380
GAG AAC AAG AGT AAA GCT GTG GAA CAG ATA AAA AAT GCC TTT 422
AAC AAG CTG CAG GAA AAA GGA ATT TAC AAA GCC ATG AGT GAA 464
TTT GAC ATT TTT ATT AAC TAC ATA GAA GCA TAC ATG ACA ATT 506
AAA GCC AGG TGA G 519

## Claims

1. The use of interleukin-10 in the manufacture of a medicament for treating tumors in a patient.

2. The use of Claim 1 wherein said interleukin-10 is selected from the group consisting of viral interleukin-10 and human interleukin-10.

3. The use of Claim 1 wherein said medicament is suitable for administration of said IL-10 by continuous infusion.

4. The use of Claim 3 wherein said medicament is suitable for administration of said IL-10 in an amount in the range of about 50-800 µg per day.

## Patentansprüche

1. Verwendung von Interleukin-10 bei der Herstellung eines Arzneimittels zum Behandeln von Tumoren in einem Patienten.

2. Verwendung von Anspruch 1, wobei das Interleukin-10 aus der Gruppe ausgewählt ist, die aus Virus-Interleukin-10 und Human-Interleukin-10 besteht.

3. Verwendung von Anspruch 1, wobei das Arzneimittel zur Verabreichung des IL-10 durch kontinuierliche Infusion geeignet ist.

4. Verwendung von Anspruch 3, wobei das Arzneimittel zur Verabreichung des IL-10 in einer Menge im Bereich von etwa 50-800 µg je Tag geeignet ist.

## Revendications

1. Utilisation de l'interleukine-10 dans la fabrication d'un médicament pour le traitement des tumeurs chez un patient.

2. Utilisation de la revendication 1 où ladite interleukine-10 est choisie dans le groupe consistant en interleukine-10 virale et interleukine-10 humaine.

3. Utilisation de la revendication 1 où ledit médicament est approprié à l'administration de ladite IL-10 par perfusion continue.

4. Utilisation de la revendication 3 où ledit médicament est approprié à une administration de ladite IL-10 en une quantité comprise entre environ 50 et 800µg par jour.
